# FASCICULE DE BREVET EUROPEEN

(11) **EP 2 811 951 B1**
(45) Date de publication et mention de la délivrance du brevet: **09.11.2016**
(21) Numéro de dépôt: 13703584.6
(22) Date de dépôt: 08.02.2013
(51) Int. Cl.: A61F 9/00

(54) **DISPOSITIF DE CONDITIONNEMENT ET DE DISTRIBUTION D'UN PRODUIT À USAGE OPHTALMIQUE**
VORRICHTUNG ZUR VERPACKUNG UND ABGABE EINER SUBSTANZ ZUR OPHTHALMISCHEN ANWENDUNG
DEVICE FOR PACKAGING AND DISPENSING A SUBSTANCE FOR OPHTHALMIC USE

(30) Priorité: 09.02.2012 FR 1251246
(43) Date de publication de la demande: 17.12.2014
(73) Titulaire: Horus Pharma, 06700 Saint-Laurent Du Var (FR)
(72) Inventeur: CLARET, Martine, F-06100 Nice (FR); ROY, Pierre, F-75019 Paris (FR)
(74) Mandataire: Regimbeau
(86) Numéro de dépôt international: PCT/EP2013/052587
(87) Numéro de publication internationale: WO 2013/117721

(56) Documents cités:
- WO-A1-2009/073482
- FR-A1- 2 873 358
- FR-A1- 2 912 998
- FR-A1- 2 941 682
- US-A1- 2008 181 930
- US-A1- 2010 226 962

## Description

### Domaine de l'invention

L'invention concerne un dispositif de conditionnement et de distribution de gouttes d'un produit à usage ophtalmique, généralement fluide, semi-fluide ou en suspension, émulsion ou solution huileuse.

### Etat de l'art

Il existe des dispositifs de conditionnement et de distribution de structure classique qui permettent de conserver et distribuer un produit sous forme de doses ou de gouttes ou sous toute autre forme, tout en maintenant sa propreté ou sa stérilité pendant toute la durée de son utilisation, sans l'adjonction de conservateurs.

Ces dispositifs sont utilisés notamment dans les domaines pharmaceutique, cosmétique, et de l'alimentation, et pour certains plus particulièrement dans le domaine ophtalmologique.

Dans la cadre de l'ophtalmologie, la délivrance d'une ou plusieurs gouttes de produit pour une grande majorité de ce type de dispositifs s'effectue en appuyant, entre le pouce et l'index, la bouteille contenant le produit.

La plupart des solutions ophtalmiques, quelle que soit leur fonction (traitement d'une maladie oculaire, cicatrisation, hydratation, etc.), sont commercialisées dans un flacon de conditionnement en Polyéthylène équipé d'un embout compte-gouttes pour leur distribution directement dans l'oeil.

Tous les flacons de ce type posent un problème de protection contre la prolifération microbienne, qui risque d'entraîner, au moment de la distribution des gouttes, la contamination microbiologique de l'oeil du patient.

Pour y remédier, il est classique d'utiliser des agents conservateurs antimicrobiens que l'on introduit en mélange dans la solution. Mais de tels agents, tel que par exemple le chlorure de benzalkonium, ont le grave inconvénient d'être agressifs pour l'oeil.

Plusieurs solutions de flacons ont été développés où sont en cours de développement permettant d'éviter l'utilisation de conservateurs en préservant la stérilité du produit au cours de son utilisation.

Les solutions décrites font appel soit à un filtre antibactérien, soit à des systèmes comportant des valves à billes et ressorts, soit à des matériaux antibactériens, soit plus particulièrement à des valves en élastomère.

Le document FR 2873358 décrit un dispositif dans lequel le produit est expulsé par un embout souple dont le récipient est muni. L'embout souple forme un petit réservoir appelé chambre de dosage et situé entre deux valves anti-retour. Une première valve est placée au niveau du réservoir ou flacon de médicament et s'ouvre en direction de la chambre de dosage. La deuxième valve, de forme annulaire, est placée après la chambre de dosage et s'ouvre en direction de l'extrémité de distribution de l'embout. Sous l'effet de la pression créée par un appui sur les parois de l'embout souple au niveau de la chambre de dosage, le liquide est expulsé vers l'ouverture du dispositif à travers la deuxième valve. Au moment du relâchement de la pression d'appui, sous l'effet de son élasticité, la chambre de dosage reprend sa forme initiale et ouvre la première valve en permettant à une nouvelle dose de produit de remplir la chambre de dosage, tandis que la seconde valve se referme. Le dispositif comporte en outre une valve au niveau du filtre d'entrée d'air. Ces valves sont réalisées en silicone.

Pour cette dernière solution, les élastomères de silicone sont souvent retenus en raison de leurs propriétés reconnues pour un usage pharmaceutique (matériaux inscrits à la pharmacopée).

Les analogues de la prostaglandine sont des médicaments utilisés exclusivement en ophtalmologie pour le traitement du glaucome, maladie souvent liée à une pression intraoculaire (PIO) trop élevée.

Une prostaglandine en particulier facilite l'écoulement de l'humeur aqueuse par le flux uvéoscléral. Des recherches ont pu mettre au point une molécule qui reproduit cet effet de façon locale. Ces médicaments ont été commercialisés pour la première fois en 1996. Latanoprost (Xalatan, société Pfizer) était la première prostaglandine développée pour le traitement du glaucome et s'avère efficace pour réduire la PIO avec une application quotidienne prise au coucher. Normalement durant la journée l'humeur aqueuse s'écoule majoritairement à travers le trabéculum et un peu par la voie uvéosclérale. Cependant, pendant la nuit elle emprunte majoritairement la voie uvéosclérale. À faible dose, le latanoprost augmente l'évacuation par la voie uvéosclérale pour une longue période et alors ne nécessite qu'une seule application quotidienne.

D'autres médicaments analogues de la prostaglandine de la même catégorie sont le travoprost (Travatan, société Alcon), le bimatoprost (Lumigan, société Allergan) et le tafluprost (Taflotan, société Santen).

Il a été constaté que dans un dispositif de conditionnement et de distribution de produit tel que décrit dans le document FR873358, le produit à usage ophtalmique comprenant un analogue de la prostaglandine n'est pas stable. En particulier, sa composition est fortement modifiée et sa concentration en analogue de la prostaglandine diminue.

Il n'existe pas dans l'art antérieur de dispositif de conditionnement et de distribution de gouttes de produit à usage ophtalmique comprenant un analogue de la prostaglandine.

Il n'existe en particulier pas dans l'art antérieur de dispositif de conditionnement de distribution de gouttes de produit à usage ophtalmique comprenant un analogue de la prostaglandine sans conservateur et assurant la stérilité du produit.

Il n'existe pas de tel dispositif qui assure la stabilité d'un tel produit. WO2009/073482A1 décrit un système distributeur de dose mesurée. US2008/0181930A1 décrit un dispositif oculaire libérant un médicament.

### Présentation générale de l'invention

L'invention est décrite par la revendication 1. Un but de l'invention est de fournir un accessoire de distribution pour un dispositif de conditionnement et de distribution d'un produit à usage ophtalmique, en particulier un produit intervenant dans le cadre d'un traitement contre le glaucome comprenant un analogue de la prostaglandine, un produit comprenant un corticoïde ou un produit comprenant un anti-inflammatoire non stéroïdien, qui permette de distribuer des gouttes de produit propre ou stérile de manière fréquente et répétitive, et qui assure un meilleur conditionnement du produit.

A cet effet, il est prévu un dispositif de conditionnement et de distribution de gouttes d'un produit à usage ophtalmique, généralement fluide, semi-fluide ou en suspension, émulsion ou solution huileuse,

comprenant un accessoire de distribution comprenant un premier clapet en un matériau élastomère permettant le passage du produit lorsque l'accessoire de distribution est sollicité sans permettre à un air extérieur de passer dans le sens inverse lorsque l'accessoire de distribution est relâché, caractérisé en ce que le premier clapet est recouvert d'une couche de parylène.

L'invention est avantageusement complétée par les caractéristiques suivantes, prises seules ou en une quelconque de leurs combinaisons techniquement possibles :
- un récipient destiné à contenir le produit et à le distribuer, l'accessoire de distribution étant monté sur le récipient et le premier clapet permettant la sortie du produit du récipient lorsque l'accessoire de distribution est sollicité sans permettre à un air extérieur d'entrer dans le récipient lorsque l'accessoire de distribution est relâché,
- un ensemble de renouvellement et de filtration de l'air entrant dans le récipient après une distribution d'une portion ou dose de produit, comprenant un second clapet en un matériau élastomère et recouvert d'une couche de parylène, permettant à l'air extérieur d'entrer dans ledit récipient lorsque l'accessoire de distribution est relâché sans permettre au produit ni sensiblement à l'air contenu à l'intérieur du récipient de sortir lorsque l'accessoire de distribution est sollicité,
- le second clapet a une forme constituée :
   o d'une forme circulaire sensiblement plane présentant deux faces, une première face étant tournée vers l'intérieur du récipient et délimitée par un rebord extérieur, et
   o d'une protubérance s'étendant sur une zone centrale de la forme circulaire délimitée par un diamètre intérieur, en prise avec la forme circulaire, s'étendant perpendiculairement à la forme circulaire de part et d'autre de la forme circulaire,
- le premier clapet a une forme de chapeau, comprenant :
   o une forme cylindrique centrale, et
   o une protubérance circonférentielle périphérique,
- le premier et/ou second clapet(s) présente(nt) une forme générale circulaire sensiblement plane délimitée par un rebord extérieur et est(sont) adapté(s) pour permettre le passage du produit :
   o par déformation au niveau d'une zone centrale, le rebord extérieur du clapet étant maintenu en position par des forces appliquées de part et d'autre de celui-ci, ou
   o par déformation du rebord extérieur, une zone centrale du clapet étant maintenue en position par des forces appliquées de part et d'autre de celui-ci,
- l'épaisseur du premier et/ou second clapet(s) croît entre la zone centrale et le rebord extérieur,
- le premier et/ou second clapet(s) au repos a un profil courbe et/ou présentant au moins un angle,
- la forme du premier et/ou second clapet(s) présente au moins une protubérance circonférentielle s'étendant sur au moins une des faces du premier et/ou du second clapet(s),
- le premier clapet présente une forme annulaire sensiblement plane délimitée par :
   o un diamètre intérieur entre 5 et 10 mm et
   o un diamètre extérieur compris entre 11 et 20 mm, le diamètre extérieur présentant une épaisseur (e) comprise entre 0,25 et 2,5 mm,
- le matériau élastomère du premier et/ou second clapet(s) est du silicone,
- le matériau élastomère du premier et/ou second clapet(s) est un élastomère thermoplastique à base polyoléfine, ou un élastomère thermoplastique à base styrène ou un polyuréthane thermoplastique,
- l'accessoire de distribution est un accessoire de distribution de gouttes calibrées comprenant :
   o une membrane souple dont la sollicitation permet de provoquer la distribution du produit,
   o une chambre de dosage du produit délimitée au moins par :
      ▪ une première portion d'une périphérie d'un noyau rigide et
      ▪ une partie de membrane souple déformable élastiquement située en regard de la première portion, la membrane souple s'étendant autour du noyau rigide, le premier clapet en un matériau élastomère permettant le passage du produit dans la chambre de dosage lorsque la membrane souple est sollicitée sans permettre à un air extérieur de passer dans le sens inverse lorsque la membrane souple est relâchée,
- des moyens de maintien en forme de la membrane souple, par rapport à au moins une deuxième portion de la périphérie du noyau rigide, les moyens de maintien en forme comprenant :
   o au moins une pièce rigide solidaire de la membrane souple et s'étendant en regard de la deuxième portion de la périphérie du noyau rigide, la pièce rigide étant située entre la membrane souple et la deuxième portion de la périphérie du noyau rigide ou
   o une pièce rigide solidaire d'une partie de la membrane souple s'étendant en regard de la deuxième portion de la périphérie du noyau rigide, la partie de la membrane souple étant située entre la pièce rigide et la deuxième portion de la périphérie du noyau rigide, ou
   o un assemblage de la membrane souple avec une surface de la deuxième portion de la périphérie du noyau rigide par collage ou soudage,
- la chambre de dosage présente en section une forme générale trapézoïdale, et/ou
- la partie de la membrane souple délimitant la chambre de dosage forme une pièce généralement prismatique qui comporte deux extrémités présentant chacune une zone de plus faible épaisseur.

L'invention concerne également une utilisation d'un tel dispositif pour le conditionnement et la distribution de gouttes calibrées d'un produit à usage ophtalmique, en particulier comprenant un analogue de la prostaglandine, un corticoïde ou un anti-inflammatoire non stéroïdien.

L'invention concerne en outre un procédé de fabrication d'un tel dispositif, comprenant :
- le moulage d'un premier et/ou second clapet(s) en élastomère,
- le traitement au parylène du premier et/ou second clapet(s) en élastomère pour couvrir sa surface d'un revêtement en parylène
- le montage du dispositif avec le premier et/ou second clapet(s) ainsi obtenu(s)

### Présentation des figures

D'autres caractéristiques et avantages de l'invention apparaitront lors de la description ci-après d'un mode de réalisation. Aux dessins annexés :
- la figure 1a, respectivement 1b, est une vue en coupe longitudinale illustrant un premier, respectivement deuxième, mode de réalisation du dispositif de conditionnement et de distribution de produit à usage ophtalmique comportant un accessoire de distribution selon l'invention ;
- la figure 2 est une vue en trois dimensions d'une pièce centrale formant noyau rigide de l'accessoire de distribution des figures 1a et 1b ;
- la figure 3a, respectivement 3b, est une vue en trois dimensions d'une membrane souple de l'accessoire de distribution illustrant le premier, respectivement deuxième, mode de réalisation du dispositif selon l'invention ;
- la figure 4a, respectivement 4b, est une vue en coupe longitudinale de la membrane souple de la figure 3a, respectivement 3b ;
- la figure 5, est une vue en trois dimensions d'une pièce externe formant une deuxième pièce rigide illustrant le premier mode de réalisation du dispositif selon l'invention ;
- la figure 6a, est une vue en coupe longitudinale de la pièce externe de la figure 5 ;
- la figure 6b est une vue en coupe longitudinale de la pièce externe formant une deuxième pièce rigide illustrant le deuxième mode de réalisation du dispositif selon l'invention ;
- la figure 7 est une vue partielle en coupe longitudinale illustrant une variante du deuxième mode de réalisation de du dispositif selon l'invention ;
- la figure 8 est une vue partielle en coupe longitudinale d'un accessoire de distribution illustrant un troisième mode de réalisation d'un dispositif selon l'invention ;
- la figure 9a, est une vue en coupe selon une section de la membrane souple de la figure 3a, au niveau de la chambre de dosage ;
- la figure 9b est une vue en coupe selon une section de l'accessoire de distribution illustrant le deuxième mode de réalisation du dispositif selon l'invention.
- les figures 10a à 10c sont des schémas présentant la déformation de chambre de dosage de l'accessoire de distribution illustrant le dispositif selon l'invention ;
- les figures 11 a à 11 c représentent schématiquement plusieurs variantes de formes de remplissage de chambre de dosage de l'accessoire de distribution illustrant le dispositif selon l'invention,
- les figures 12a à 12h représentent schématiquement plusieurs variantes de formes de premier clapet illustrant le dispositif selon l'invention,
- les figures 13a à 13g représentent schématiquement plusieurs variantes de formes de second clapet illustrant le dispositif selon l'invention.
- les figures 14a à 14c et 15a à 15c représentent des résultats d'analyse par chromatographie concernant un clapet selon l'art antérieur et un clapet illustrant un dispositif selon l'invention.

### Description de l'invention

### Exemples illustrant des modes de réalisation du dispositif

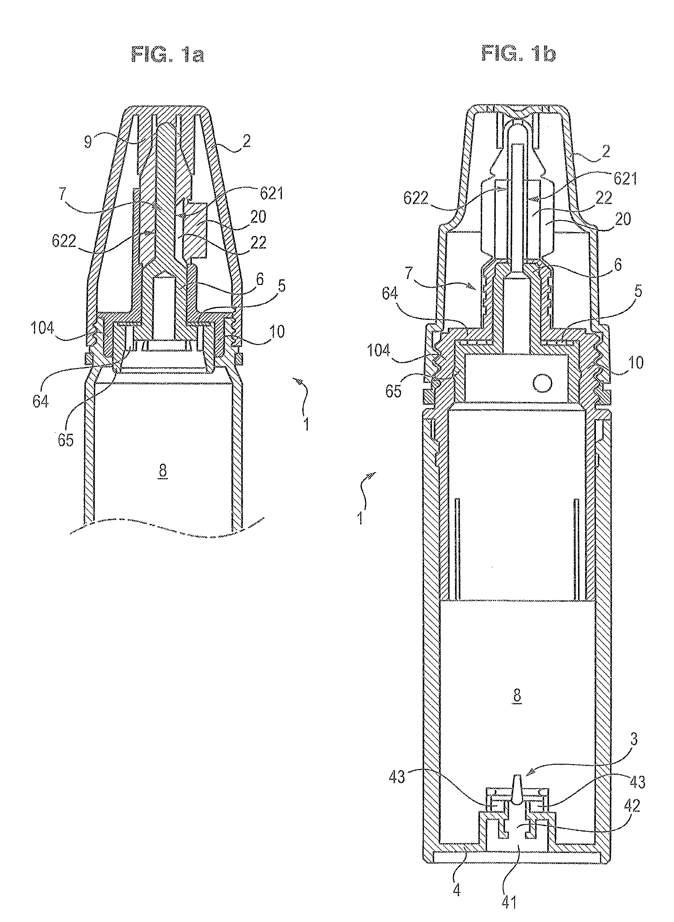

En référence aux figures 1a et 1b, il est décrit un dispositif de conditionnement et de distribution de produit à usage ophtalmique 1 comportant un accessoire de distribution 7. La figure 1a, respectivement 1b, illustre un premier, respectivement un deuxième, mode de réalisation d'un dispositif. Les caractéristiques de ces deux modes de réalisation peuvent être combinées.

Le dispositif de conditionnement et de distribution 1 comprend un récipient 8. Le récipient 8 est fermé à l'une de ses extrémités par un fond 4 comportant des moyens d'entrée et de filtration d'air. Ces moyens d'entrée et de filtration d'air permettent d'introduire de l'air dans l'enceinte du récipient 8 au fur et à mesure que le produit initialement contenu dans ladite enceinte est distribué. L'air entrant étant alors filtré, ce qui permet d'assurer le maintien de la propreté ou de la stérilité du produit restant dans l'enceinte du récipient 8.

De tels moyens sont, par exemple décrits, plus en détail dans le document FR 2772007.

A une extrémité opposée de celle comportant le fond 4, le récipient 8 présente une ouverture ainsi que l'accessoire de distribution 7 venant obstruer ladite ouverture. L'accessoire de distribution 7, et illustrant le premier, respectivement deuxième mode de réalisation de la figure 1a, respectivement 1 b, comporte une pièce centrale formant un noyau rigide 6 sur laquelle vient s'emboiter de manière coaxiale une membrane souple 20, une deuxième pièce rigide 10 comportant au moins une excroissance 101, ainsi qu'un premier clapet 5. Un bouchon 2 amovible complète le dispositif de conditionnement et de distribution 1 en recouvrant l'accessoire de distribution 7 afin de le protéger lorsque le dispositif 1 n'est pas utilisé.

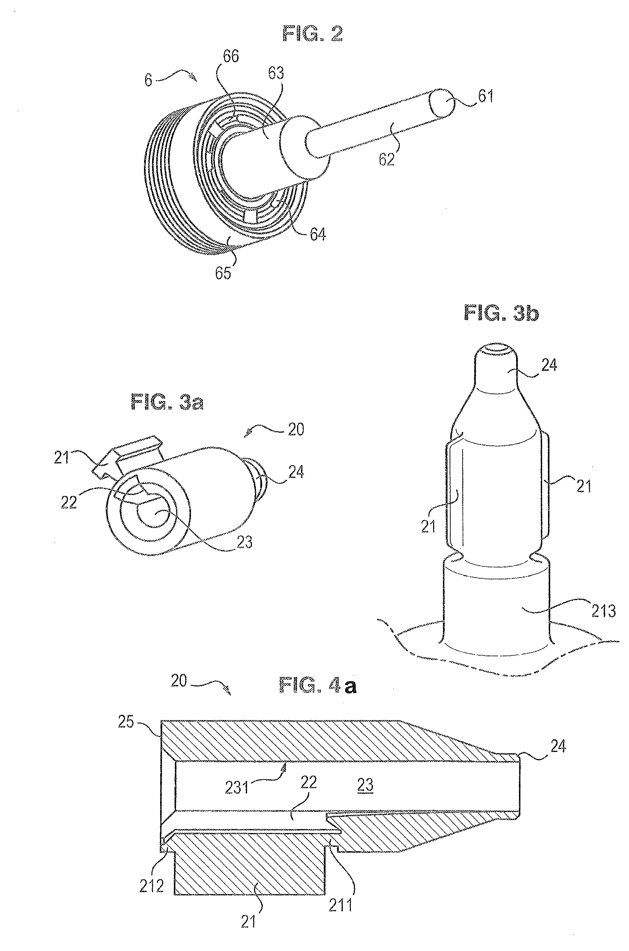

Le noyau rigide 6, représenté sur la figure 2 en trois dimensions, s'étend de manière longitudinale et comporte à une extrémité une embase 65 ouverte vers l'enceinte du récipient 8 lorsque ladite embase 65 est montée de manière étanche dans l'ouverture dudit récipient 8. Un épaulement 66 relie l'embase 65 à un premier tronçon 63. L'épaulement 66 comporte une cavité annulaire au fond de laquelle des orifices 64 traversant sont aménagés. Ici, les orifices 64 sont au nombre de quatre et uniformément répartis sur une circonférence. Le nombre ainsi que la forme de ces orifices 64 peuvent varier et être adaptés au produit à usage ophtalmique qui est destiné à être contenu dans le récipient 8 et à être distribué à l'aide de l'accessoire de distribution 7. Les orifices 64 forment un passage entre l'intérieur de l'embase 65 et l'extérieur. Le premier tronçon 63 se prolonge longitudinalement par un deuxième tronçon 62 de section plus petite qu'une section du premier tronçon 63. Le deuxième tronçon 62 s'étend longitudinalement jusqu'à une extrémité opposée 61 du noyau rigide 6 par rapport à l'embase 65.

### Exemples de membrane souple

La membrane souple 20 illustrant le premier mode de réalisation est représentée figure 3a selon une vue en trois dimensions et figure 4a selon une vue en coupe. Elle est de forme sensiblement cylindrique et s'étend longitudinalement entre une extrémité 25 et une extrémité 24. La membrane souple 20 comporte un évidement 23 coaxial de forme cylindrique et s'étendant longitudinalement entre les deux extrémités 24 et 25.

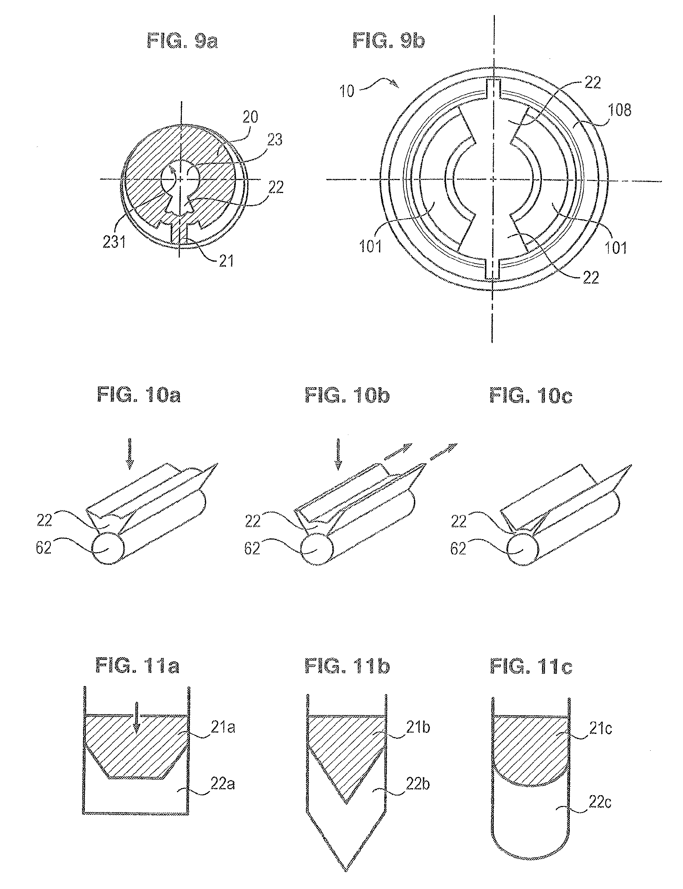

De plus, s'étendant depuis l'extrémité 25 sur environ une première moitié, la membrane souple 20 comporte au moins une chambre 22 dite de dosage aménagée dans une épaisseur de la paroi de la membrane et ouverte vers l'évidement 23. En regard de cette chambre de dosage 22, l'évidement 23 comporte une surface 231. En référence à la figure 9a, la chambre de dosage 22 présente, en section, une forme globalement trapézoïdale dont une petite base forme l'ouverture dans l'évidement 23 qui s'étend alors le long d'une première portion d'une périphérie de l'évidement 23. La surface 231 en regard s'étend sur une deuxième portion de la périphérie de l'évidement 23 complémentaire de la première portion de la périphérie dudit évidement 23.

De manière centrifuge, la chambre de dosage 22 est délimitée par une partie 21 de la membrane souple. Cette partie 21 forme une pièce généralement de forme prismatique. Elle est déformable élastiquement et comporte à chacune de ses extrémités une zone de plus faible épaisseur 211, 212. Ainsi, cela permet, lors d'un appui sur la partie 21, un déplacement quasi-vertical de la pièce prismatique dans la chambre de dosage 22 et de réduire les efforts à fournir lors d'une mise en oeuvre du dispositif pour distribuer une dose ou goutte de produit contenu dans l'enceinte du récipient 8.

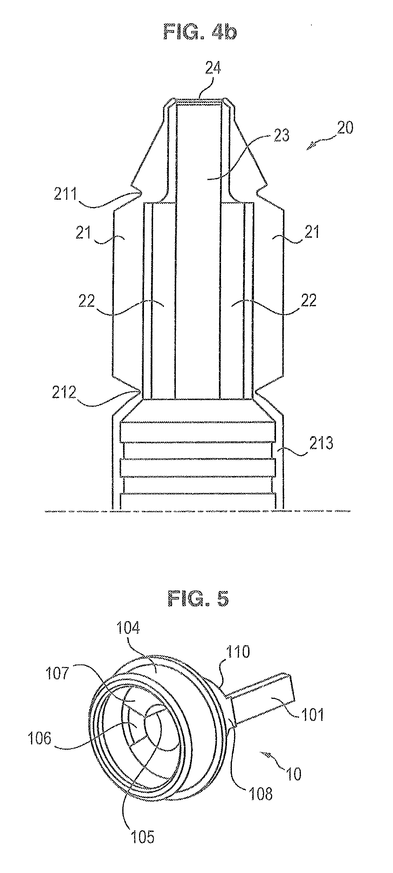

La membrane souple 20 illustrant le deuxième mode de réalisation est représentée figure 3b selon une vue en trois dimensions, figure 4b selon une vue en coupe. Elle se différencie de la membrane souple illustrant le premier mode de réalisation en ce qu'elle comprend deux chambres de dosage 22 diamétralement opposées. La deuxième pièce rigide 10 est à l'intérieur de la membrane souple 20 qui est, au montage, emmanchée sur le noyau rigide 6 et sur la deuxième pièce 10. La membrane souple 20 comporte une jupe 213 recouvrant une partie intermédiaire 108 de la deuxième pièce 10. L'excroissance 101 est alors prise en sandwich entre la membrane souple 20 et une portion du deuxième tronçon 62 du noyau rigide 6. La membrane souple 20 est collée ou soudée sur une surface externe de l'excroissance 101 ce qui permet qu'elle ne puisse pas se déformer lors d'une utilisation.

Par ailleurs, entre les zones 211 et 212, l'évidement 23 présente un diamètre plus important qu'entre la zone 211 et l'extrémité 24. La zone correspondant aux chambres de dosage 22 est donc comprise dans l'évidement 23. Comme dans le premier mode de réalisation, les chambres de dosage 22 sont toujours limitées d'une part par la partie 21 et d'autre part par une portion du deuxième tronçon 62. Cependant les deux autres parois s'étendant longitudinalement de la chambre de dosage sont désormais constituées par des portions de parois latérales de deux excroissances 101 de la deuxième pièce rigide 10.

### Exemples de deuxième pièce rigide

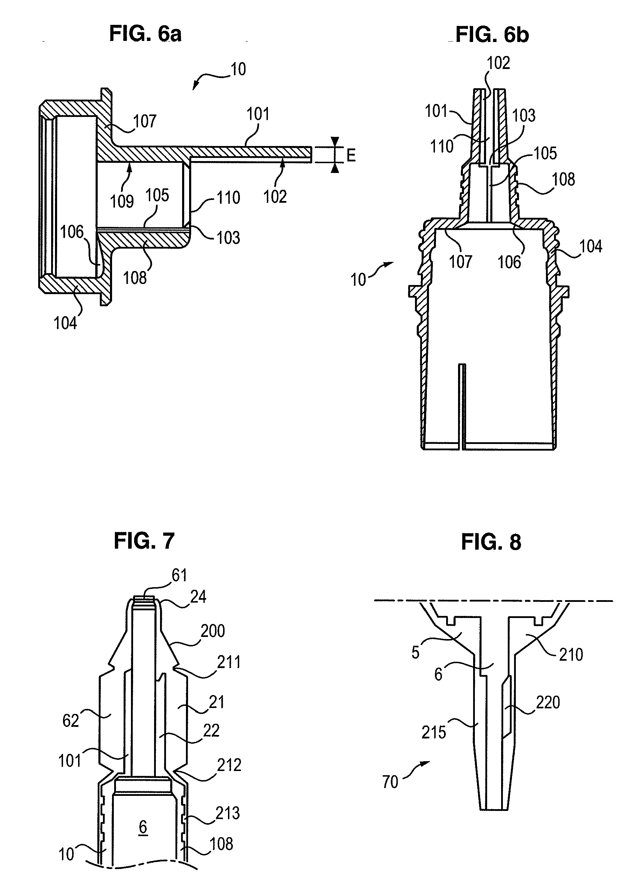

La deuxième pièce rigide 10 illustrant le premier mode de réalisation, représentée aux figures 5 et 6a, comporte au niveau d'une première extrémité une base 104, ouverte du côté de la première extrémité. Cette base 104 est surmontée dans le sens longitudinale d'une partie intermédiaire 108 creuse, reliée à la base 104 par un épaulement 107. Pour chaque chambre de dosage 22, l'épaulement 107 comporte, sur une face interne, un creux 106 dont une ouverture est orientée vers l'intérieur de la base 104 de sorte, en outre, à déboucher dans un conduit 105 aménagé de manière longitudinale sur une paroi interne 109 de la partie intermédiaire 108. La partie intermédiaire 108 se termine par une ouverture 103 formant une deuxième extrémité 110 de la deuxième pièce rigide 10 et au niveau de laquelle débouche le conduit 105. L'ouverture 103 et le conduit 105 permettent de relier chaque chambre de dosage 22 au creux 106 dédié. Une excroissance 101 rigide s'étend de manière longitudinale en saillie depuis la deuxième extrémité 110. L'excroissance 101 comporte une face interne 102 de forme concave semi-circulaire. L'excroissance peut être de forme en section en arc de cercle présentant une épaisseur E comprise entre 0,5 et 1,5 mm. L'excroissance 101 est située de manière diamétralement opposée par rapport au conduit 105.

La deuxième pièce rigide 10 selon le deuxième mode de réalisation est illustrée à la figure 6b. Elle présente deux excroissances 101 rigides s'étendant de manière longitudinale en saillie depuis la deuxième extrémité 110. Les excroissances 101 comportent une face interne 102 de forme concave semi circulaire. Les excroissances 101 sont de forme en section en arc de cercle présentant une épaisseur E. Les chambres de dosages 22 s'étendent dans les deux sections annulaires situées entre les excroissances 101. Des faces longitudinales de largeur E des excroissances 101 constituent des parois des chambres de dosage 22.

### Exemple d'assemblage de l'accessoire de distribution

La figure 1a représente un assemblage de l'accessoire de distribution 7 illustrant un premier mode de réalisation du dispositif. Dans la cavité annulaire de l'épaulement 66 du noyau rigide 6 est placé un premier clapet 5, ici réalisé en matériau élastomérique souple déformable élastiquement recouvert d'une couche de parylène, venant recouvrir les orifices 64. Puis, la deuxième pièce rigide 10 est emmanchée à coulissement sur le noyau rigide 6 de sorte que la base 104 de la deuxième pièce rigide 10 vienne recouvrir l'embase 65 du noyau rigide 6. Une fois en position, le premier clapet 5 se trouve pris en sandwich entre les épaulements 66 et 107 du noyau rigide 6 et de la deuxième pièce rigide 10 respectivement. En utilisation, le premier clapet 5 peut s'ouvrir par déformation dans le creux 106 de la deuxième pièce rigide 10, ce qui dégage un passage depuis au moins l'un des orifices 64 du noyau rigide 6. L'emmanchement ainsi réalisé est étanche car la base 104 est complémentaire de l'embase 65, de même que la partie intermédiaire 108 de la deuxième pièce rigide est complémentaire du premier tronçon 63 du noyau rigide 6. Le maintien en place de la deuxième pièce rigide 10 et du noyau rigide 6 est assuré par clipsage, collage et/ou soudage.

La membrane souple 20 est ensuite emmanchée à coulissement sur le deuxième tronçon 62 du noyau rigide 6 et l'excroissance 101 de la deuxième pièce rigide 10, jusqu'à ce que l'extrémité 25 vienne en contact sur la deuxième extrémité 110 de la partie intermédiaire 108 de la deuxième pièce rigide 10 et sur un sommet du premier tronçon 63 du noyau rigide 6. Dès lors, l'au moins une chambre de dosage 22 est délimitée par la partie 21 de la membrane souple 20 et par une première portion 621 de la périphérie du deuxième tronçon 62. La surface 231 de la membrane souple est en contact avec une deuxième portion 622 de la périphérie du noyau rigide, deuxième portion 622 complémentaire de la première portion 621. D'autre part, dans, le cas illustré ici, la membrane souple 20 est prise longitudinalement en sandwich entre l'excroissance 101 de la deuxième pièce rigide 10, dont la face interne 102 est en appui sur une surface externe de la membrane souple, et le deuxième tronçon 62 du noyau rigide 6. Ainsi, la surface 231 de la membrane souple est en contact permanent avec la deuxième portion 622 de la périphérie du noyau rigide. Au surplus, la face interne 102 peut être collée ou soudée sur la surface externe de la membrane souple 20. Ainsi, l'excroissance 101 s'étend sur une distance équivalente à une dimension longitudinale de la chambre de dosage 22 et est située de manière diamétralement opposée à ladite chambre de dosage. De plus, le conduit 105 débouche aussi dans la chambre de dosage 22 afin de l'alimenter suite à une distribution par ladite chambre de dosage d'une dose ou goutte de produit à distribuer.

L'accessoire de distribution 7 ainsi assemblé est ensuite monté de manière étanche sur l'ouverture du récipient 8. L'assemblage de l'accessoire de distribution 7 illustrant le deuxième mode de réalisation du dispositif se réalise de manière équivalente, aux particularités de la deuxième pièce rigide 10 et de la membrane souple 20 près. Ainsi la membrane souple 20 vient couvrir la partie intermédiaire 108 de la deuxième pièce rigide 10.

Dans les illustrations des deux modes de réalisation du dispositif, au repos, l'extrémité 24 de la membrane souple 20 est en appui étanche avec une surface de l'extrémité 61 du noyau rigide 6. Lors d'un appui sur la partie 21 de la membrane souple 20, le produit contenu dans la chambre de dosage 22 correspondante est mis sous pression. Du fait de la présence du premier clapet 5, le produit à distribuer ne peut retourner dans l'enceinte du récipient 8. L'extrémité 24 de la membrane souple se déforme alors en se décollant la surface de l'extrémité 61 du noyau rigide 6, laissant ainsi sortir pour distribution ledit produit à distribuer. Une fois le produit expulsé, l'extrémité 24 revient en position de repos sur l'extrémité 61. Le relâchement de l'appui sur la partie 21 de la membrane souple 20 permet à cette partie 21 de revenir en position de repos, créant une dépression dans la chambre de dosage qui cherche à reprendre sa forme de repos. Comme le contact entre l'extrémité 24 de la membrane souple 20 avec l'extrémité 61 du noyau rigide forme un clapet anti-retour (empêchant l'air et les contaminants externes de pénétrer dans la chambre de dosage), le clapet 5 s'ouvre afin de laisser passer du produit à distribuer depuis l'enceinte du récipient 8 dans la chambre de dosage 22 via le conduit 5.

### Exemples illustrant des modes de réalisations des premier et second clapets

### Exemples de premier clapet

Le premier clapet 5 est réalisé par une première étape de moulage en matériau élastomère, et par une deuxième étape de traitement du clapet au parylène afin de recouvrir sa surface d'un revêtement en parylène, préférentiellement du parylène C.

Le matériau élastomère peut être du silicone. Le matériau élastomère peut être un élastomère thermoplastique à base polyoléfine, tel que l'éthylène-propylène-diène-monomère. Le matériau élastomère peut être un élastomère thermoplastique à base styrène, par exemple styrène-butadiène ou styrène-éthylène-butylène ou styrène-éthylène-propylène. Le matériau peut être un polyuréthane thermoplastique.

Le premier clapet 5 présente une forme générale annulaire sensiblement plane d'un diamètre extérieur D dont la valeur est comprise par exemple entre 11 et 20 mm, par exemple entre 13 et 17 mm, par exemple 15 mm. La forme annulaire du premier clapet 5 est en outre délimitée par un rebord intérieur 53 d'un diamètre intérieur d compris entre 5 et 10 mm, par exemple entre 6,5 et 8,5 mm, par exemple 7,5 mm. L'épaisseur du premier clapet 5 peut être comprise par exemple entre 0,25 et 2,5 mm, par exemple 0,5 et 1,5 mm.

La forme du premier clapet 5 présente au moins une protubérance circonférentielle s'étendant sur au moins une des faces du premier clapet 5.

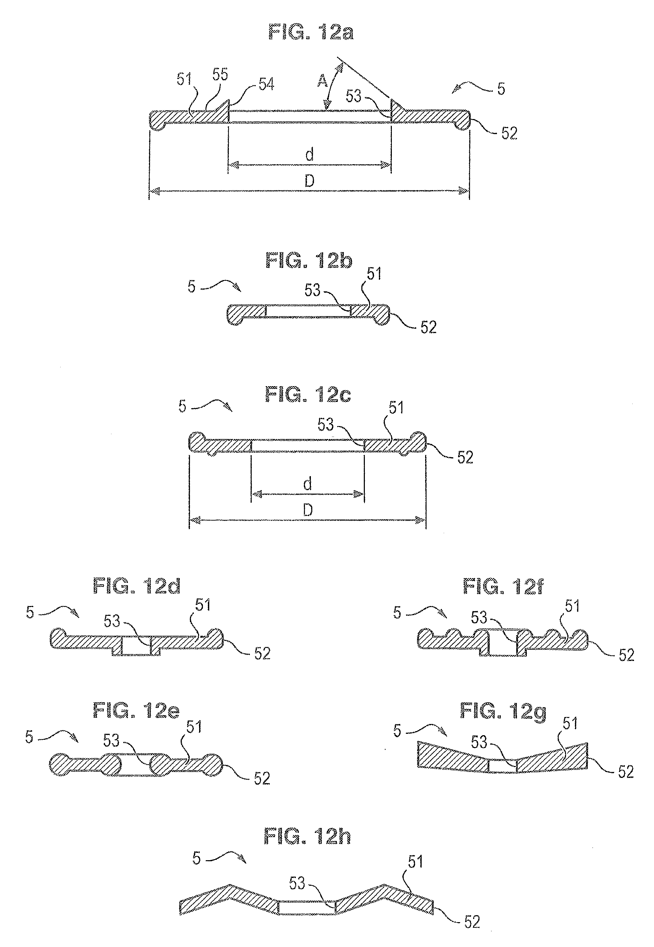

Les exemples de premier clapet 5 représentés sur les figures 12a à 12g sont adaptés pour permettre le passage du produit à usage ophtalmique par déformation au niveau d'une ouverture centrale, un rebord extérieur 52 du clapet 5 étant maintenu en position par des forces appliquées de part et d'autre de celui-ci. Un tel fonctionnement est illustré par les figures relatives au deuxième mode de réalisation.

La figure 12a illustre une forme annulaire 51 présentant une protubérance circonférentielle périphérique convexe au niveau du rebord extérieur 52 du premier clapet 5, d'une épaisseur maximale de 0,5 mm, sur une face du premier clapet 5. La forme présente en outre une protubérance circonférentielle centrale au niveau du rebord intérieur 53, d'une épaisseur maximale de 0,5 mm, sur une face opposée du premier clapet 5. Le diamètre extérieur D est de 15 mm. Le diamètre intérieur d est de 7,5 mm. L'épaisseur de la forme annulaire 51 est de 0,5 mm. La protubérance circonférentielle centrale comprend une surface annulaire 54 d'un diamètre extérieur D de 9 mm, en périphérie de laquelle se trouve une zone de transition 55 oblique annulaire présentant un angle A de 45° par exemple.

La figure 12b illustre une forme annulaire 51 présentant uniquement une protubérance circonférentielle périphérique convexe au niveau du rebord extérieur 52.

La figure 12c illustre une forme annulaire 51 présentant une protubérance circonférentielle convexe périphérique de 0,5 mm de rayon s'étendant sur une face du premier clapet 5, au niveau du rebord extérieur 52, et une protubérance circonférentielle convexe intermédiaire de 0,25 mm de rayon s'étendant sur la face opposée du premier clapet 5, à distance du rebord intérieur 53 et du rebord extérieur 52.

La figure 12d illustre une forme annulaire 51 présentant une protubérance circonférentielle convexe périphérique sur une face du premier clapet 5, au niveau du rebord extérieur 52, et une protubérance circonférentielle centrale, au niveau du rebord intérieur 53, sur la face opposée du premier clapet 5.

La figure 12e illustre une forme annulaire 51 présentant une protubérance circonférentielle convexe périphérique sur les deux faces du premier clapet 5, au niveau du rebord extérieur 52, et une protubérance circonférentielle centrale s'étendant sur les deux faces du premier clapet 5, au niveau du rebord intérieur 53.

La figure 12f illustre une forme annulaire 51 présentant une protubérance circonférentielle convexe périphérique s'étendant sur une face du premier clapet, au niveau du rebord extérieur 52, une protubérance circonférentielle centrale s'étendant sur les deux faces du premier clapet 5, au niveau du rebord intérieur 53, et une protubérance circonférentielle convexe intermédiaire s'étendant sur une face du premier clapet 5, à distance des rebords intérieur 53 et extérieur 52.

La figure 12g illustre une forme annulaire 51 présentant une épaisseur croissante entre le rebord intérieur 53 et le rebord extérieur 52.

Les exemples de premier clapet 5 également représentés sur les figures 12a à 12g, ainsi que la figure 12h sont adaptés pour permettre le passage du produit par déformation du rebord extérieur 52, une zone centrale du premier clapet 5 au niveau du rebord intérieur 53 étant maintenue en position par des forces appliquées de part et d'autre de celui-ci.

La figure 12h illustre une forme annulaire 51 d'épaisseur constante dont un profil au repos présente un angle.

Selon un autre exemple, le premier clapet 5 peut avoir une forme de chapeau, comprenant une forme cylindrique centrale, et une protubérance circonférentielle périphérique. L'accessoire de distribution 7 peut alors comprendre un élément de rappel tel qu'un ressort pour maintenir le premier clapet dans une position de blocage. Selon un tel exemple, l'accessoire de distribution 7 ne comprend pas nécessairement de chambre de dosage.

### Exemples de second clapet

La figure 1b représente un exemple de moyens d'entrée et de filtration d'air illustrant le deuxième mode de réalisation du dispositif. L'air passe par une ouverture du fond 4, et traverse un filtre tubulaire 41. A la sortie du filtre 41 se trouve une chambre 42 cylindrique dont une ouverture est close par une zone centrale d'un second clapet 3 comprenant un matériau élastomère, recouvert d'une couche de parylène.

Le matériau élastomère peut être du silicone. Le matériau élastomère peut être un élastomère thermoplastique à base polyoléfine, tel que l'éthylène-propylène-diène-monomère. Le matériau élastomère peut être un élastomère thermoplastique à base styrène, par exemple styrène-butadiène ou styrène-éthylène-butylène ou styrène-éthylène-propylène. Le matériau peut être un polyuréthane thermoplastique.

Le second clapet 3 permet à l'air extérieur d'entrer dans le récipient 8 lorsque la membrane souple 20 de l'accessoire de distribution 7 est relâchée sans permettre au produit ni sensiblement à l'air contenu à l'intérieur du récipient 8 de sortir lorsque la membrane souple 20 est sollicitée.

Le second clapet 3 présente une forme générale circulaire sensiblement plane délimitée par un rebord extérieur et est adapté pour permettre le passage du produit par déformation au niveau d'une ouverture centrale, le rebord extérieur du second clapet 3 étant maintenu en position par des forces appliquées de part et d'autre de celui-ci. Le rebord extérieur est muni de dents ou de fentes. Lorsque la zone centrale du second clapet 3 se déforme, l'air situé dans la chambre 42 peut entrer dans une deuxième chambre 43 annulaire et située en périphérie de la première chambre 42. Cette chambre 43 est directement connectée à l'intérieur du récipient 8 par les interstices entre les dents du rebord extérieur ou les fentes du rebord extérieur.

De manière alternative, le second clapet 3 peut permettre le passage de l'air extérieur filtré par déformation du rebord extérieur, la zone centrale du clapet 3 étant maintenue en position par des forces appliquées de part et d'autre de celui-ci. Dans ce cas, la première chambre 42 est sensiblement annulaire.

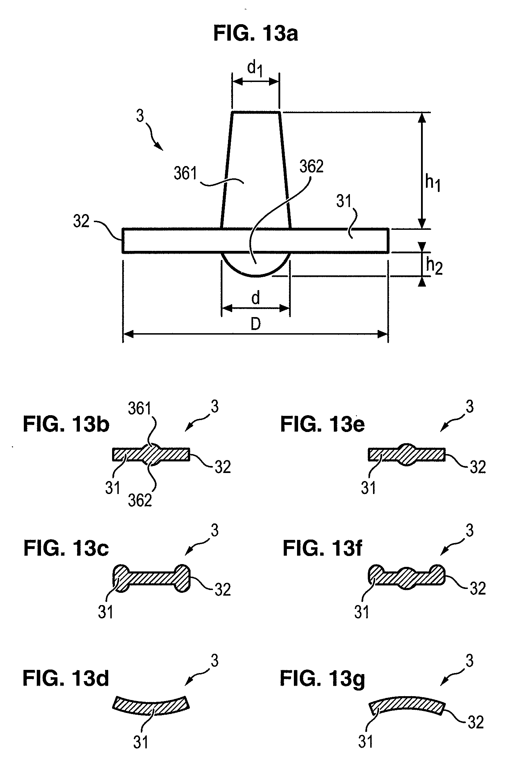

Les figures 13a à 13c illustrent des exemples de second clapet 3 permettant le passage de l'air par déformation de la zone centrale du second clapet 3.

Le second clapet 3 illustré figure 13a présente une forme constituée d'une forme circulaire 31 et d'une protubérance 361 et 362. La forme circulaire 31 est sensiblement plane et présente deux faces, une première face et une deuxième face. La première face est tournée vers l'intérieur du récipient 8. La forme circulaire 31 est délimitée par un diamètre extérieur D. Le diamètre extérieur D peut prendre une valeur comprise par exemple entre 3 et 9 mm, Le diamètre extérieur D peut prendre une valeur comprise par exemple entre 5 et 6 mm, par exemple 5,70 mm. La protubérance s'étend sur une zone centrale de la forme circulaire 31 définie par un diamètre intérieur d. Le diamètre intérieur d peut prendre une valeur comprise entre 1 et 3 mm, par exemple 1,5 mm. La protubérance en prise avec la forme circulaire 31, s'étendant perpendiculairement à la forme circulaire 31 de part et d'autre de la forme circulaire 31 en une première protubérance 361 en prise avec la première face et une deuxième protubérance 362 en prise avec la deuxième face. La première protubérance 361 est de section circulaire. Elle peut présenter une hauteur h1 comprise entre 1 et 5 mm, par exemple entre 2 et 3 mm, par exemple 2,5 mm. L'extrémité de la première protubérance peut être constituée d'une surface circulaire d'un diamètre d1 compris entre 0,5 et 1 mm, par exemple 1 mm. La deuxième protubérance 362 peut être convexe, d'une hauteur h2 comprise entre 0,25 et 1 mm, par exemple 0,5 mm.

La figure 13b illustre un exemple de second clapet 3 qui présente une première protubérance 361 et une deuxième protubérance 362 toutes deux de forme convexe.

La figure 13c illustre un exemple de second clapet 3 de forme circulaire présentant sur chaque face une protubérance circonférentielle convexe au niveau du rebord extérieur.

La figure 13d illustre un exemple de second clapet 3 présentant au repos un profil courbe, en forme d'arc de cercle la première face étant du côté convexe de la courbe et la deuxième face étant du côté concave de la courbe.

Les figures 13e à 13g illustrent des exemples de second clapet 3 permettant le passage de l'air par déformation du rebord extérieur du second clapet 3.

La figure 13e illustre un exemple de second clapet 3 qui présente une première protubérance 361 et une deuxième protubérance 362 toutes deux de forme convexe.

La figure 13f illustre un exemple de second clapet 3 qui présente une première protubérance 361 et une deuxième protubérance 362 toutes deux de forme convexe, ainsi que sur chaque face une protubérance circonférentielle convexe au niveau du rebord extérieur.

La figure 13g illustre un exemple de second clapet 3 présentant au repos un profil courbe, en forme d'arc de cercle la première face étant du côté concave de la courbe et la deuxième face étant du côté convexe de la courbe.

### Traitement au parylène

Des analyses ont mis en évidence une interaction forte entre les analogues de la prostaglandine et les composants des dispositifs de distributions de conditionnement et de distribution de gouttes calibrées d'un produit à usage ophtalmique.

Il a été montré par des tests que l'interaction forte concernait les clapets en élastomère de silicone et les analogues de la prostaglandine. Le déposant a constaté que l'interaction se caractérisait par une adsorption ou absorption de la prostaglandine par l'élastomère, en particulier le silicone.

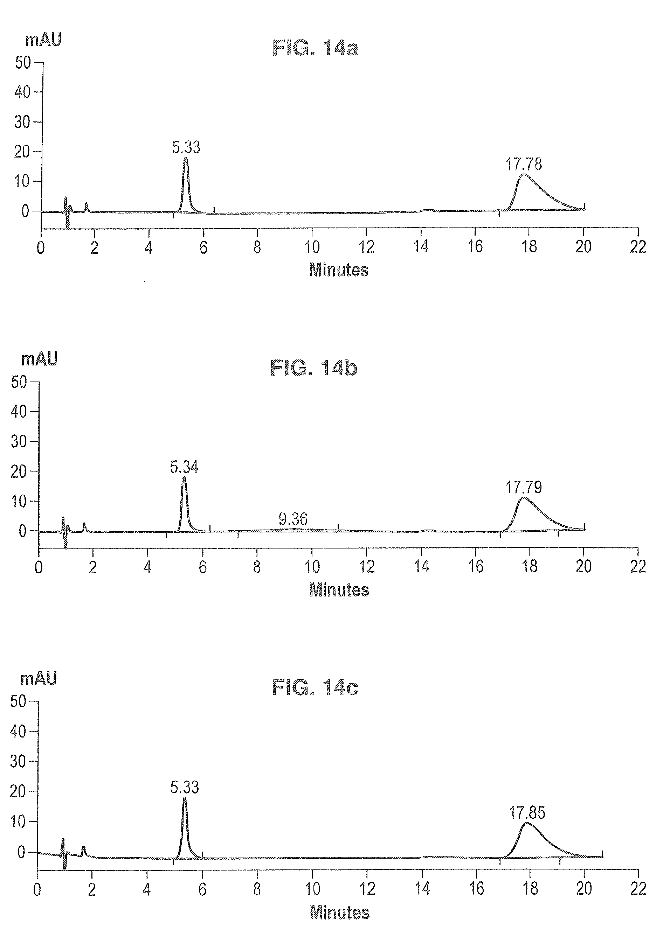

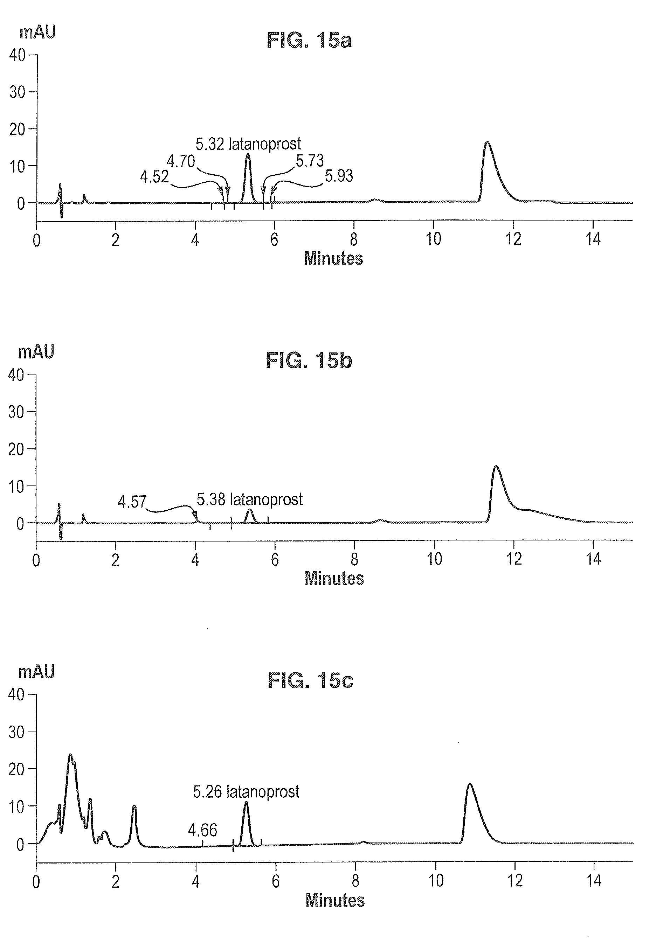

Comme l'illustrent les figures 14a à 14c et 15a à 15c, il a été observé qu'un traitement au poly-p-xylylène, ou parylène, consistant à revêtir les clapets en élastomère d'une couche de parylène permettait de résoudre ce problème posé par les membranes en silicone ou en fluorosilicone. Le parylène, présente des propriétés d'étanchéité en couche mince et biocompatible.

Cette observation résulte d'analyses par chromatographie liquide haute performance réalisées sur des solutions ophtalmiques à base de Latanoprost. Les analyses impliquent un appareillage comprenant un sytème LACHROM ELITE. L'appareillage comprend des pompes L2130, un four L2300, un injecteur L2200, un détecteur L2400 et un logiciel LACHROM ELITE. La colonne est de type Nucleosil 100-5-C18 (125 x 4) - 5 µm Réf MN 721622-40. L'éluant consiste en KH₂PO₄ 0,05 M (6,8 g/l) ajusté à pH = 3 avec du H₃PO₄ (pour 50 V) et en acétonitrile (pour 50 V). Le débit pratiqué est de 1,0 mL.min⁻¹. La détection a pour niveau 210 nm. Le volume injecté est de 5 µL. Le temps de rétention pratiqué pour le Latanoprost est d'environ 4,4 minutes.

Les figures 14a, respectivement 14b et 14c présentent une mesure par chromatographie présentant des à l'état initial une solution de Latanoprost, respectivement d'une solution de Latanoprost et d'une matrice d'éléments de clapet en élastomère selon l'art antérieur, et d'une solution de Latanoprost et d'une matrice d'éléments de clapet en élastomère couverts d'une couche de parylène. Le pic caractéristique du Latanoprost se situe à 5,34 minutes. Les figures 15a, 15b et 15c présentent les résultats d'analyse par chromatographie liquide haute performance des mêmes solutions après trente jours. Les résultats d'analyses des solutions testées par chromatographie liquide haute performance en termes de teneur en latanoprost en ppm à l'instant initial, à dix jours et à trente jours sont consignés dans la table 1.

**TABLE 1**

| | **T=0** | **T = 10 jours** | **T = 30 jours** |
|---|---|---|---|
| **Latanoprost + matrice art antérieur** | 48,1 ppm | 24,2 ppm | 15,2 ppm |
| **Latanoprost + matrice traité au parylène** | 49,4 ppm | 48,2 ppm | 45,1 ppm |

Le traitement au parylène permet de résoudre des problèmes similaires d'interactions entre les clapets et certains corticoïdes ainsi que certains anti-inflammatoires non stéroïdiens que peut contenir le produit à usage ophtalmique du dispositif.

Les clapets décrits dans le document FR 2873358 sont de formes circulaires et parfaitement plates. Ils ne présentent pas de courbure, d'angle, ou de protubérance. Ces clapets posent des problèmes d'étanchéité du dispositif. Par ailleurs, ces clapets adhèrent fortement entre eux et s'attachent les uns aux autres, ce qui pose des problèmes d'industrialisation, en particulier au sujet de leur stockage et de leur manipulation. La présence de reliefs et de formes non planes difficiles à emboîter sont des exemples de formes ne présentant pas de tels inconvénients. Le traitement subséquent au parylène permet également de diminuer les propriétés d'adhérence de la silicone.

Le traitement au parylène permet de résoudre des problèmes similaires d'interactions entre un produit à usage ophtalmique et les clapets en un autre matériau élastomère tel qu'un élastomère thermoplastique à base polyoléfine, par exemple l'éthylène-propylène-diène-monomère, ou un élastomère thermoplastique à base styrène, par exemple styrène-butadiène ou styrène-éthylène-butylène ou styrène-éthylène-propylène, ou un polyuréthane thermoplastique.

Le traitement au parylène permet également de diminuer les propriétés d'adhérence de ces élastomères.

### Exemples de modes de réalisation de la chambre de dosage

Le fait que l'accessoire de distribution 7 comporte des moyens de maintien en contact permanent (l'excroissance 101, respectivement les excroissances 101 de la deuxième pièce rigide 10 du dispositif illustrant le premier, respectivement deuxième, mode de réalisation de l'invention) de la membrane souple 20 avec la deuxième portion 622 de la périphérie du noyau rigide 6 permet d'assurer que l'ensemble du volume de produit contenu dans la chambre de dosage 22 soit bien éjecté vers l'extrémité 61 du noyau rigide 6, qui fait alors office d'embout de distribution. En effet, l'écrasement d'un cylindre de matière élastomérique implique lors de sa déformation, une augmentation dans le sens radial du rayon initial. L'étanchéité étant nécessaire à l'expulsion du liquide situé dans la chambre de dosage, toute fuite entraîne un dysfonctionnement du système. Dans le cas d'espèce, si la membrane n'était pas maintenu en contact avec le noyau rigide, comme indiqué ci-dessus, un appui sur la partie 21 de ladite membrane conduirait à une déformation de la membrane (sous l'effet de l'écrasement et de la montée en pression du produit alors présent dans la chambre) qui s'éloignerait alors de la deuxième portion 622 de la périphérie : une partie de la dose de produit à distribuer contenu dans la chambre de dosage s'infiltrerait dans l'interstice ainsi créé et ne serait pas expulsé au niveau de l'extrémité 24 de la membrane souple 20. Une dose incorrecte serait alors fournie au patient. L'excroissance 101 de la deuxième pièce rigide 10, située alors en opposition à la chambre de dosage 22 limite ces déformations.

En figure 7, illustrant une variante du deuxième mode de réalisation du dispositif est illustré, cette variante comprenant des caractéristiques des dispositifs illustrant le premier mode de réalisation en ce qu'elle ne comprend qu'une unique excroissance 101. Ici, la deuxième pièce rigide 10 est à l'intérieur de la membrane souple 20 qui est, au montage, emmanchée sur le noyau rigide 6 et sur la deuxième pièce 10. La membrane souple 20 comporte une jupe 213 recouvrant la partie intermédiaire 108 de la deuxième pièce 10. L'unique excroissance 101 est alors prise en sandwich entre la membrane souple 20 et le deuxième tronçon 62 du noyau rigide 6. La membrane souple 20 est collée ou soudée sur une surface externe de l'excroissance 101 ce qui permet qu'elle ne puisse pas se déformer lors d'une utilisation.

En une autre variante de réalisation, la membrane souple est au moins surmoulée sur l'excroissance 101 de la deuxième pièce rigide.

Dans un troisième mode de réalisation d'un dispositif, illustré à la figure 8 et dont seules les différences avec le premier mode de réalisation vont être décrites, l'accessoire de distribution 7 ne comporte pas de deuxième pièce rigide 10. La membrane souple 20 est directement emmanchée sur le noyau rigide 6, emprisonnant le clapet 5 dans l'embase du noyau rigide. Les déformations de la membrane souple 20 sont empêchées lors d'une utilisation de distribution, par collage ou soudage de la partie 215 de la membrane souple 20 sur la deuxième portion 622 de la périphérie du noyau rigide 6.

D'un point de vue ergonomique, il résulte des documents de l'art antérieur une grande imprécision du geste qui entraîne soit de toucher la surface oculaire avec l'extrémité de l'embout, donc un risque de contamination de celui-ci, soit de ne pas atteindre la surface oculaire, d'où une mauvaise observance du traitement. La force nécessaire pour l'actionner peut en outre constituer un inconvénient supplémentaire.

Les figures 9a et 9b illustrent le fonctionnement de chaque chambre de dosage 22. Du fait de la nature élastomérique de la membrane souple 20, la forme en section de la chambre de dosage 22 est généralement trapézoïdale, donnant à la chambre de dosage 22 la forme d'un prisme, ainsi la partie 21 de la membrane souple 20 présente une forme générale prismatique qui, lors d'un appui, remplit de manière optimale la chambre de dosage 22. Cette forme a de plus l'avantage, dans son fonctionnement, d'être indépendante de la viscosité du produit à distribuer expulsé de ladite chambre, tout en offrant une déformation minimale de la partie 21. Au fur et à mesure de l'appui, l'intervalle entre la partie 21 externe au prisme formant la chambre de dosage 22 et ladite chambre de dosage 22 se réduit, ce qui permet d'augmenter le cisaillement du produit à distribuer contenu dans la chambre de dosage 22, donc d'expulser les liquides visqueux, dont la viscosité dépend du taux de cisaillement. Certains produits pharmaceutiques, sont des solutions visqueuses dont la viscosité dépend du taux de cisaillement, par exemple une composition dont la loi de comportement viscoélastique décroit en fonction du l'intensité du cisaillement et qui a pour objet de reproduire le comportement du fluide lacrymal de viscosité élevée au repos et faible sous l'influence du cisaillement, provoqué par exemple par le clignement des paupières, à l'inverse des fluides de type Newtoniens de viscosité constante.

Pour un fluide Newtonien, la viscosité restera constante quelle que soit le taux de cisaillement, ainsi, la force utilisée pour évacuer le produit de la chambre de dosage 22 sera proportionnelle à la surface de chambre en contact avec le liquide, c'est à dire élevée au début de l'appui et diminuant progressivement en relation avec la surface en contact.

A l'inverse, lorsque le produit à usage ophtalmique est un fluide rhéofluidifiant, de viscosité élevée à faible cisaillement, comme décrit par EP0698388 dans le cas d'une solution hyaluronique destinée à des larmes artificielles, cette force sera encore plus élevée au début de l'appui, il convient donc de proposer une chambre de dosage 22 permettant de diminuer cette force initiale.

Pour une chambre de dosage 22 comportant une partie fixe et une partie mobile, la zone de point de contact entre la partie fixe et la partie mobile se présente sous la forme d'un entrefer s'élargissant progressivement entre l'épaisseur nulle au point de contact et une épaisseur donnée, de préférence faible. Ainsi, lors d'une sollicitation A de la partie mobile, le liquide sera le plus fortement cisaillé aux abords du point de contact, donc sera de plus faible viscosité et s'écoulera E de façon préférentielle dans cette zone. Cela est illustré aux figures 10a à 10c.

Ainsi, dans les premier et deuxième modes de réalisation du dispositif , la membrane souple 20, au moins au niveau de sa portion constituant au moins une partie de la chambre de dosage 22, est réalisée dans un matériau élastomère, se déformant sous l'action d'un appui A, et présentant de la même façon, une zone de plus faible épaisseur au niveau du contact entre la partie mobile et la partie fixe, et de forme générale prismatique.

La forme donnée à la partie 21 venant remplir la chambre de dosage 22 peut prendre plusieurs variantes illustrées de manière non limitative par les figures 11a à 11c. Ces exemples conservent la notion d'un l'écart s'amenuisant progressivement, ce qui permet de soumettre une partie seulement du liquide à un taux de cisaillement élevé et permettre ainsi son écoulement de façon préférentielle dans la zone de cisaillement élevée et non dans l'ensemble de la chambre de dosage 22.

La figure 11a illustre schématiquement une forme plane biseautée sur les extérieures de la partie 21a venant remplir la chambre de dosage 22a.

La figure 11 b illustre schématiquement une forme en coin de la partie 21 b venant remplir la chambre de dosage 22b.

La figure 11 c illustre schématiquement une forme convexe arrondie de la partie 21 c venant remplir la chambre de dosage 22c.

## Revendications

1. Dispositif de conditionnement et de distribution de gouttes d'un produit à usage ophtalmique, généralement fluide, semi-fluide ou en suspension, émulsion ou solution huileuse, comprenant un accessoire de distribution (7) comprenant un premier clapet (5) en un matériau élastomère permettant le passage du produit lorsque l'accessoire de distribution (7) est sollicité sans permettre à un air extérieur de passer dans le sens inverse lorsque l'accessoire de distribution (7) est relâché, **caractérisé en ce que** le premier clapet (5) est recouvert d'une couche de parylène,
dans lequel le dispositif comprend :
- un récipient (8) destiné à contenir le produit et à le distribuer, l'accessoire de distribution (7) étant monté sur le récipient (8) et le premier clapet (5) permettant la sortie du produit du récipient (8) lorsque l'accessoire de distribution est sollicité sans permettre à un air extérieur d'entrer dans le récipient (8) lorsque l'accessoire de distribution (7) est relâché,
- un ensemble de renouvellement et de filtration de l'air entrant dans le récipient (8) après une distribution d'une portion ou dose de produit, comprenant un second clapet (3) en un matériau élastomère et recouvert d'une couche de parylène, permettant à l'air extérieur d'entrer dans ledit récipient (8) lorsque l'accessoire de distribution (7) est relâché sans permettre au produit ni sensiblement à l'air contenu à l'intérieur du récipient (8) de sortir lorsque l'accessoire de distribution (7) est sollicité,
le second clapet (3) ayant une forme constituée :
o d'une forme circulaire (31) sensiblement plane présentant deux faces, une première face étant tournée vers l'intérieur du récipient (8) et délimitée par un rebord extérieur (32), et
o d'une protubérance (361, 362) s'étendant sur une zone centrale de la forme circulaire (31) délimitée par un diamètre intérieur (d), en prise avec la forme circulaire (31), s'étendant perpendiculairement à la forme circulaire (31) de part et d'autre de la forme circulaire (31),
et/ou
le second (3) clapet(s) présentant une forme générale circulaire sensiblement plane (31) délimitée par un rebord extérieur (32) et étant adapté pour permettre le passage du produit :
o par déformation au niveau d'une zone centrale, le rebord extérieur (32) du clapet (3) étant maintenu en position par des forces appliquées de part et d'autre de celui-ci, ou
o par déformation du rebord extérieur (32), une zone centrale du clapet (3) étant maintenue en position par des forces appliquées de part et d'autre de celui-ci,
et/ou
dans lequel le premier (5) clapet présente une forme générale circulaire sensiblement plane (51) délimitée par un rebord extérieur (52) et est adapté pour permettre le passage du produit :
- par déformation au niveau d'une zone centrale (53), le rebord extérieur (52) du clapet (5) étant maintenu en position par des forces appliquées de part et d'autre de celui-ci, ou
- par déformation du rebord extérieur (52), une zone centrale (53) du clapet (5) étant maintenue en position par des forces appliquées de part et d'autre de celui-ci,
et/ou
dans lequel l'accessoire de distribution est un accessoire de distribution (7) de gouttes calibrées comprenant :
- une membrane souple (20) dont la sollicitation permet de provoquer la distribution du produit,
- une chambre de dosage (22) du produit délimitée au moins par :
o une première portion (621) d'une périphérie d'un noyau rigide (6) et
o une partie (21) de membrane souple (20) déformable élastiquement située en regard de la première portion (621), la membrane souple (20) s'étendant autour du noyau rigide (6),
le premier clapet (5) en un matériau élastomère permettant le passage du produit dans la chambre de dosage (22) lorsque la membrane souple (20) est sollicitée sans permettre à un air extérieur de passer dans le sens inverse lorsque la membrane souple (20) est relâchée.

2. Dispositif selon l'une des revendications précédentes, **caractérisé en ce que** le premier clapet (5) a une forme de chapeau, comprenant :
- une forme cylindrique centrale, et
- une protubérance circonférentielle périphérique.

3. Dispositif selon la revendication 1 ou 2, **caractérisé en ce que** l'épaisseur du premier (5) et/ou second (3) clapet(s) croît entre la zone centrale (53) et le rebord extérieur (52, 32).

4. Dispositif selon l'une quelconque des revendications 1 à 3, **caractérisé en ce que** le premier (5) et/ou second (3) clapet(s) au repos a un profil courbe et/ou présentant au moins un angle.

5. Dispositif selon l'une quelconque des revendications 1 à 4, **caractérisé en ce que** la forme du premier (5) et/ou second (3) clapet(s) présente au moins une protubérance circonférentielle s'étendant sur au moins une des faces du premier (5) et/ou du second (3) clapet(s).

6. Dispositif selon l'une des revendications 1 à 5, **caractérisé en ce que** le premier clapet (5) présente une forme annulaire (51) sensiblement plane délimitée par :
- un diamètre intérieur (d) entre 5 et 10 mm et
- un diamètre extérieur (D) compris entre 11 et 20 mm,
le diamètre extérieur (D) présentant une épaisseur (e) comprise entre 0,25 et 2,5 mm.

7. Dispositif selon l'une quelconque des revendications précédentes, **caractérisé en ce que** le matériau élastomère du premier (5) et/ou second (3) clapet(s) est du silicone ou un élastomère thermoplastique à base polyoléfine, ou un élastomère thermoplastique à base styrène ou un polyuréthane thermoplastique.

8. Dispositif selon l'une quelconque des revendications 1 à 7, **caractérisé en ce qu'**il comprend des moyens de maintien en forme (101) de la membrane souple (20), par rapport à au moins une deuxième portion de la périphérie du noyau rigide (6), les moyens de maintien en forme (101) comprenant :
- au moins une pièce rigide (101) solidaire de la membrane souple (20) et s'étendant en regard de la deuxième portion de la périphérie du noyau rigide (6), la pièce rigide (101) étant située entre la membrane souple (20) et la deuxième portion de la périphérie du noyau rigide (6) ou
- une pièce rigide solidaire d'une partie de la membrane souple s'étendant en regard de la deuxième portion de la périphérie du noyau rigide (6), la partie de la membrane souple (20) étant située entre la pièce rigide (101) et la deuxième portion de la périphérie du noyau rigide (6), ou
- un assemblage de la membrane souple (20) avec une surface de la deuxième portion de la périphérie du noyau rigide (6) par collage ou soudage.

9. Dispositif selon l'une quelconque des revendications 1 à 8, **caractérisé en ce que** :
- la chambre de dosage (22) présente en section une forme générale trapézoïdale, et/ou
- la partie (21) de la membrane souple (20) délimitant la chambre de dosage (22) forme une pièce généralement prismatique qui comporte deux extrémités présentant chacune une zone de plus faible épaisseur.

10. Utilisation d'un dispositif selon l'une quelconque des revendications précédentes pour le conditionnement d'un produit à usage ophtalmique comprenant un analogue de la prostaglandine ou un corticoïde ou un anti-inflammatoire non stéroïdien.

11. Procédé de fabrication d'un dispositif selon l'une quelconque des revendications 1 à 9, **caractérisé en ce qu'**il comprend :
- le moulage d'un premier (5) et/ou second (3) clapet(s) en élastomère,
- le traitement au parylène du premier (5) et/ou second (3) clapet(s) en élastomère pour couvrir sa surface d'un revêtement en parylène,
- le montage du dispositif avec le premier (5) et/ou second clapet(s) (3) ainsi obtenu(s).

## Patentansprüche

1. Vorrichtung zur Verpackung und Abgabe von Tropfen eines Produkts zur ophthalmischen Anwendung, das allgemein flüssig, halbflüssig ist oder in Suspension, Emulsion oder öliger Lösung vorliegt, umfassend ein Abgabeaccessoire (7), umfassend ein erstes Ventil (5) aus einem Elastomermaterial, das den Durchgang des Produkts erlaubt, wenn das Abgabeaccessoire (7) beansprucht wird, ohne einer Außenluft zu gestatten, in die umgekehrte Richtung zu passieren, wenn das Abgabeaccessoire (7) losgelassen wird, **dadurch gekennzeichnet, dass** das erste Ventil (5) mit einer Parylenschicht bedeckt ist,
wobei die Vorrichtung umfasst:
- einen Behälter (8), der dazu bestimmt ist, das Produkt zu enthalten und abzugeben, wobei das Abgabeaccessoire (7) auf dem Behälter (8) montiert ist und das erste Ventil (5) den Ausgang des Produkts aus dem Behälter (8) erlaubt, wenn das Abgabeaccessoire beansprucht wird, ohne einer Außenluft zu gestatten, in den Behälter (8) einzudringen, wenn das Abgabeaccessoire (7) losgelassen wird,
- eine Erneuerungs- und Filtrationseinheit der in den Behälter (8) nach einer Abgabe einer Produktportion oder -dosis eindringenden Luft, umfassend ein zweites Ventil (3) aus einem Elastomermaterial und bedeckt mit einer Parylenschicht, das der Außenluft gestattet, in den Behälter (8) einzudringen, wenn das Abgabeaccessoire (7) losgelassen wird, ohne weder dem Produkt noch spürbar der im Innern des Behälters (8) enthaltenen Luft zu erlauben zu entweichen, wenn das Abgabeaccessoire (7) beansprucht wird,
wobei das zweite Ventil (3) eine Form hat, die gebildet ist von:
o einer etwa ebenen kreisförmigen Form (31), die zwei Flächen aufweist, wobei eine erste Fläche zum Innern des Behälters (8) zeigt und von einem Außenrand (32) begrenzt ist, und
o einem Vorsprung (361, 362), der sich über eine zentrale Zone der kreisförmigen Form (31) erstreckt, begrenzt durch einen Innendurchmesser (d), in der kreisförmigen Form (31) inbegriffen, der sich senkrecht zu der kreisförmigen Form (31) beiderseits der kreisförmigen Form (31) erstreckt,
und/oder
wobei das zweite Ventil (3) eine allgemein etwa ebene kreisförmige Form (31) aufweist, die von einem Außenrand (32) begrenzt ist und ausgebildet, um den Durchgang des Produkts zu erlauben:
o durch Verformen im Bereich einer zentralen Zone, wobei der Außenrand (32) des Ventils (3) mittels beiderseits auf ihn angewendeter Kräfte in Position gehalten wird, oder
o durch Verformen des Außenrands (32), wobei eine zentrale Zone des Ventils (3) mittels der beiderseits auf ihn angewendeter Kräfte in Position gehalten wird,
und/oder
wobei das erste Ventil (5) eine allgemein etwa ebene kreisförmige Form (51) aufweist, die von einem Außenrand (52) begrenzt ist und ausgebildet, um den Durchgang des Produkts zu erlauben:
- durch Verformen im Bereich einer zentralen Zone (53), wobei der Außenrand (52) des Ventils (5) mittels beiderseits auf ihn angewendeter Kräfte in Position gehalten wird, oder
- durch Verformen des Außenrands (52), wobei eine zentrale Zone (53) des Ventils (5) mittels beiderseits auf ihn angewendeter Kräfte in Position gehalten wird,
und/oder
wobei das Abgabeaccessoire ein Abgabeaccessoire (7) kalibrierter Tropfen ist, umfassend:
- eine elastische Membran (20), deren Beanspruchung erlaubt, die Abgabe des Produkts zu bewirken,
- eine Dosierkammer (22) des Produkts, die begrenzt ist mindestens von:
o einem ersten Abschnitt (621) einer Peripherie eines festen Kerns (6) und
o einem elastisch verformbaren Teil (21) elastischer Membran (20), der sich gegenüber dem ersten Abschnitt (621) befindet, wobei sich die elastische Membran (20) um den festen Kern (6) erstreckt,
wobei das erste Ventil (5) aus einem Elastomermaterial den Durchgang des Produkts in die Dosierkammer (22) erlaubt, wenn die elastische Membran (20) beansprucht wird, ohne einer Außenluft zu gestatten, in die umgekehrte Richtung zu passieren, wenn die elastische Membran (20) losgelassen wird.

2. Vorrichtung nach einem der vorangehenden Ansprüche, **dadurch gekennzeichnet, dass** das erste Ventil (5) die Form einer Kappe hat, umfassend:
- eine zentrale zylindrische Form, und
- einen peripheren Umfangsvorsprung.

3. Vorrichtung nach Anspruch 1 oder 2, **dadurch gekennzeichnet, dass** die Dicke des ersten (5) und/oder zweiten (3) Ventils zwischen der zentralen Zone (53) und dem Außenrand (52, 32) zunimmt.

4. Vorrichtung nach einem der Ansprüche 1 bis 3, **dadurch gekennzeichnet, dass** das erste (5) und/oder zweite (3) Ventil in Ruhestellung ein gekrümmtes Profil hat/haben und/oder mindestens einen Winkel aufweist/aufweisen.

5. Vorrichtung nach einem der Ansprüche 1 bis 4, **dadurch gekennzeichnet, dass** die Form des ersten (5) und/oder zweiten (3) Ventils mindestens einen Umfangsvorsprung aufweist, der sich über mindestens eine der Flächen des ersten (5) und/oder des zweiten (3) Ventils erstreckt.

6. Vorrichtung nach einem der Ansprüche 1 bis 5, **dadurch gekennzeichnet, dass** das erste Ventil (5) eine etwa ebene ringförmige Form (51) aufweist, die begrenzt ist von:
- einem Innendurchmesser (d) zwischen 5 und 10 mm und
- einem Außendurchmesser (D) zwischen 11 und 20 mm inklusive,
wobei der Außendurchmesser (D) eine Dicke (e) zwischen 0,25 und 2,5 mm inklusive aufweist.

7. Vorrichtung nach einem der vorangehenden Ansprüche, **dadurch gekennzeichnet, dass** das Elastomermaterial des ersten (5) und/oder zweiten (3) Ventils Silikon oder ein thermoplastisches Elastomer auf der Basis von Polyolefin oder ein thermoplastisches Elastomer auf der Basis von Styrol oder ein thermoplastisches Polyurethan ist.

8. Vorrichtung nach einem der Ansprüche 1 bis 7, **dadurch gekennzeichnet, dass** sie Informhaltemittel (101) der elastischen Membran (20) in Bezug zu mindestens einem zweiten Abschnitt der Peripherie des festen Kerns (6) umfasst, wobei die Informhaltemittel (101) umfassen:
- mindestens ein starres Teil (101), das mit der elastischen Membran (20) fest verbunden ist und sich gegenüber dem zweiten Abschnitt der Peripherie des festen Kerns (6) erstreckt, wobei sich das starre Teil (101) zwischen der elastischen Membran (20) und dem zweiten Abschnitt der Peripherie des festen Kerns (6) befindet, oder
- ein starres Teil, das mit einem Teil der elastischen Membran fest verbunden ist, das sich gegenüber dem zweiten Abschnitt der Peripherie des festen Kerns (6) erstreckt, wobei sich der Teil der elastischen Membran (20) zwischen dem starren Teil (101) und dem zweiten Abschnitt der Peripherie des festen Kerns (6) befindet, oder
- eine Verbindung der elastischen Membran (20) mit einer Oberfläche des zweiten Abschnitts der Peripherie des festen Kerns (6) mittels Kleben oder Schweißen.

9. Vorrichtung nach einem der Ansprüche 1 bis 8, **dadurch gekennzeichnet, dass**:
- die Dosierkammer (22) im Querschnitt eine allgemein trapezförmige Form aufweist, und/oder
- der Teil (21) der elastischen Membran (20), welcher die Dosierkammer (22) begrenzt, ein allgemein prismatisches Teil bildet, das zwei Enden aufweist, die jeweils eine Zone geringerer Dicke aufweisen.

10. Verwendung einer Vorrichtung nach einem der vorangehenden Ansprüche für die Verpackung und die Abgabe eines Produkts zur ophthalmischen Anwendung, umfassend ein analoges Mittel des Prostaglandins oder ein Kortikoid oder ein nicht steroides Mittel gegen Entzündungen.

11. Verfahren zur Herstellung einer Vorrichtung nach einem der Ansprüche 1 bis 9, **dadurch gekennzeichnet, dass** es umfasst:
- das Formen eines ersten (5) und/oder zweiten (3) Ventils aus Elastomer,
- die Behandlung mit Parylen des ersten (5) und/oder zweiten (3) Ventils aus Elastomer, um seine Oberfläche mit einer Parylenbeschichtung zu bedecken,
- die Montage der Vorrichtung mit dem derart erhaltenen ersten (5) und/oder zweiten Ventil (3).

## Claims

1. A device for packaging and dispensing drops of a product for ophthalmic use, generally fluid, semi-fluid or in suspension, emulsion or oily solution, comprising a dispensing accessory (7) comprising a first valve (5) made of elastomer material allowing passage of the product when the dispensing accessory (7) is stressed without allowing outside air to move in the reverse direction when the dispensing accessory (7) is released, **characterized in that** the first valve (5) is covered with a layer of parylene,
wherein the device comprises:
- a container (8) intended to contain the product and dispense it, the dispensing accessory (7) being mounted on the container (8) and the first valve (5) allowing the product to exit the container (8) when the dispensing accessory is stressed without allowing outside air to enter the container (8) when the dispensing accessory (7) is released,
- a renewal and filtration assembly of the air entering the container (8) after dispensing of a product portion or dose, comprising a second valve (3) made of elastomer material and covered with a layer of parylene, allowing outside air to enter said container (8) when the dispensing accessory (7) is released without allowing the product or substantially the air contained inside the container (8) to exit when the dispensing accessory (7) is stressed,
the second valve (3) having a shape consisting of:
o a substantially plane circular shape (31) having two faces, a first face being turned towards the interior of the container (8) and delimited by an outer rim (32), and
o a protuberance (361, 362) extending over a central area of the circular shape (31) delimited by an inner diameter (d), engaged with the circular shape (31), extending perpendicularly to the circular shape (31) on either side of the circular shape (31),
and/or
the second (3) valve having a substantially plane general circular shape (31) delimited by an outer rim (32) and is adapted to allow passage of the product:
o by deformation at the level of a central area, the outer rim (32) of the valve (3) being held in position by forces applied on either side of the latter, or
o by deformation of the outer rim (32), a central area of the valve (3) being held in position by forces applied on either side of the latter,
and/or
wherein the first (5) valve has a substantially plane general circular shape (51) delimited by an outer rim (52) and is adapted to allow passage of the product:
- by deformation at the level of a central area (53), the outer rim (52) of the valve (5) being held in position by forces applied on either side of the latter, or
- by deformation of the outer rim (52), a central area (53) of the valve (5) being held in position by forces applied on either side of the latter,
and/or
wherein the dispensing accessory is a dispensing accessory (7) for calibrated drops comprising:
- a flexible membrane (20) whereof the stress causes dispensing of the product,
- a metering chamber (22) of the product delimited at least by:
o a first portion (621) of a periphery of a rigid core (6) and
o a part (21) of elastically deformable flexible membrane (20) located facing the first portion (621), the flexible membrane (20) extending about the rigid core (6),
the first valve (5) made of elastomer material allowing the product to enter the metering chamber (22) when the flexible membrane (20) is stressed without allowing outside air to move in the reverse direction when the flexible membrane (20) is released.

2. The device according to one of the preceding claims, **characterized in that** the first valve (5) has a hat shape, comprising:
- a central cylindrical shape, and
- a peripheral circumferential protuberance.

3. The device according to claim 1 or 2, **characterized in that** the thickness of the first (5) and/or second (3) valve(s) increases between the central area (53) and the outer rim (52, 32).

4. The device according to any one of claims 1 to 3, **characterized in that** the first (5) and/or second (3) valve(s) at rest has a curve profile and/or has at least one angle.

5. The device according to any one of claims 1 to 4, **characterized in that** the shape of the first (5) and/or second (3) valve(s) has at least one circumferential protuberance extending over at least one of the faces of the first (5) and/or of the second (3) valve(s).

6. The device according to one of claims 1 to 5, **characterized in that** the first valve (5) has a substantially plane annular shape (51) delimited by:
- an inner diameter (d) between 5 and 10 mm and
- an outer diameter (D) of between 11 and 20 mm, the outer diameter (D) having a thickness (e) of between 0.25 and 2.5 mm.

7. The device according to any one of the preceding claims, **characterized in that** the elastomer material of the first (5) and/or second (3) valve(s) is silicone or a thermoplastic elastomer with polyolefin base, or a styrene-based thermoplastic elastomer or a thermoplastic polyurethane.

8. The device according to any one of claims 1 to 7, **characterized in that** it comprises shape-holding means (101) of the flexible membrane (20), relative to at least one second portion of the periphery of the rigid core (6), the shape-holding means (101) comprising:
- at least one rigid piece (101) integral with the flexible membrane (20) and extending facing the second portion of the periphery of the rigid core (6), the rigid piece (101) being located between the flexible membrane (20) and the second portion of the periphery of the rigid core (6), or
- a rigid piece integral with a part of the flexible membrane extending facing the second portion of the periphery of the rigid core (6), the part of the flexible membrane (20) being located between the rigid piece (101) and the second portion of the periphery of the rigid core (6), or
- an assembly of the flexible membrane (20) with a surface of the second portion of the periphery of the rigid core (6) by adhesion or welding.

9. The device according to any one of claims 1 to 8, **characterized in that**:
- the metering chamber (22) has a general trapezoid shape in cross-section, and/or
- the part (21) of the flexible membrane (20) delimiting the metering chamber (22) forms a generally prismatic piece which comprises two ends each having an area of lesser thickness.

10. Use of the device according to any one of the preceding claims for packaging of a product for ophthalmic use comprising an analog of prostaglandin or a corticoid or a non-steroidal anti-inflammatory.

11. Method for manufacturing a device according to any one of claims 1 to 9, **characterized in that** it comprises:
- moulding of a first (5) and/or second (3) valve(s) made of elastomer,
- parylene processing of the first (5) and/or second (3) valve(s) made of elastomer for covering its surface with a coating of parylene,
- mounting of the device with the resulting first (5) and/or second valve(s) (3).
